# EUROPEAN PATENT APPLICATION

(11) **EP 4 101 443 A1**
(43) Date of publication of application: **14.12.2022**
(21) Application number: 21178007.7
(22) Date of filing: 07.06.2021
(51) Int. Cl.: A61K 9/50, A61K 31/616

(54) **HOT-MELT COATED PARTICLES WITH PH-DEPENDENT RELEASE**

(71) Applicant: HERMES PHARMA GmbH, 82049 Pullach (DE)
(72) Inventor: SALAR BEHZADI, Sharareh, 1080 Vienna (AT); ZIMMER, Andreas, 8010 Graz (AT); SCHERTEL, Sonja, 82049 Pullach (DE)
(74) Representative: Pharma Patents International AG

(57) **Abstract**

The invention provides a hot-melt coated pH-dependent-release particle comprising (i) a core comprising, or consisting of, an active ingredient, and (ii) a coating comprising, or consisting of, a solid fatty acid and a citric acid ester of mono- and diglycerides of fatty acids (so-called CITREM ester). The pH-dependent release may optionally be an enteric release according to pharmacopoeial requirements. The invention further provides a method for the preparation of the hot-melt coated pH-dependent release particle, as well as pharmaceutical or nutraceutical compositions comprising said particle or a plurality thereof.

## Description

### BACKGROUND OF THE INVENTION

The invention relates to coated particles with pH-dependent release, which allow only small amounts of the drug contained within the coated particles, such as no more than 25 %, to be released at low pH-values (i.e., in acidic media such as in the stomach), while ensuring a faster release at elevated pH-values (i.e., in basic media such as in the intestines). This kind of pH-dependent release is particularly suited, for instance, (i) for drugs which are sensitive to the gastric environment, either in terms of enzymes and/or its acidity, such as most proton-pump inhibitors (PPIs), enzymes like lactase, or pancreatin, or antibiotics like erythromycin; (ii) for drugs which irritate the gastric mucosa such as iron-salts, zinc-salts, or most non-steroidal anti-inflammatory drugs (NSAIDs); (iii) for drugs exhibiting a narrow absorption window in the upper intestines which should thus preferably be delivered there as a concentrated 'bulk'; and/or (iv) drugs which otherwise benefit from an initial lag-time of about 2-4 h in their release.

By preventing acid-related effects that are detrimental to the absorption of the active ingredient, such as premature activation and thus poor absorbability of proton-pump inhibitors, and by concentrating the 'bulk' being released in the (upper) intestines, pH-dependent release can also improve the overall bioavailability of the respective active ingredients. By preventing excessive contact of the gastro-irritant active ingredient with the gastric mucosa, stomach irritations, nausea, and in the worst case, ulcers can be prevented, or at least significantly limited.

However, the approach of pH-dependent release can be equally suited for masking drugs exhibiting undesirable organoleptic properties, such as poor taste (bitter, sour, metallic, hot, etc.), poor smell, or similarly unpleasant sensations such as burning, stinging, or astringent mouthfeel. For instance, pH-dependent release can help avoid the unpleasant 'smelly burps' after oral ingestion of compositions comprising strong smelling oils such as fish oils.

There exist a number of polymers, typically ionizable polymers, that can be used as enteric coating substances because they only dissolve at pHs between about 4.8 and about 7.2 or higher, such as hydroxypropyl methylcellulose phthalate (HPMCP), cellulose acetate phthalate (CAP), cellulose acetate trimellitate (CAT), or methacrylic acid copolymers like Eudragit^{®} L or Eudragit^{®} R (polymethacrylate copolymers with acidic groups that allow them to not get dissolved in the stomach yet, and thereby limit drug release there, but subsequently becoming soluble in the intestines at pH-values ≥ 5.5 or ≥ 7.0, respectively). Unfortunately, these polymers typically require lengthy coating processes using either aqueous dispersions of the polymers or solutions thereof in organic solvents.

Working with aqueous polymer dispersions, while common, usually requires lots of time for drying of the water in said dispersions (often about 70 % of the dispersion), and furthermore requires the use of plasticizers in many applications to ensure that the solid polymer-particles coalesce into a coherent, closed film-coating. Some aqueous dispersion coatings also require curing (e.g., thermal curing) after the actual coating process to prevent late storage instabilities; said thermal curing can be particularly critical for temperature-sensitive drugs. There is also a risk, especially for drugs with high water solubility (e.g., drugs with an aqueous solubility of about ≤ 35 mg/mL at a temperature of 15-25 °C), that the drug is partially dissolved into the applied coating during the aqueous coating step, potentially leading to poor taste-masking, premature drug release, and/or an increased risk of microbial contamination due to the hydrophilic drug retaining excess moisture within the core.

Organic-solvent-based coatings, on the other hand, may be quicker to apply and suffer less from storage-effects but are more expensive and far less environmentally friendly than aqueous ones, and can even be hazardous if not recovered and recycled properly.

Furthermore, some of the polymers used for achieving pH-dependent release, or specifically enteric release, in pharmaceutical products may not be suited or approved for use in nutraceutical products. For instance, not all synthetic or semi-synthetic enteric polymers are 'food-grade' approved, and/or their advised daily intake (ADI) values are not compatible with common nutraceuticals. Hence, a number of attempts have been documented in the prior art to overcome issues with the present approaches of pH-dependent release from both pharmaceutical and nutraceutical products.

For instance, Patil et al. suggest hot-melt coating (HMC) inter alia as a means to avoid the use of organic solvents and as a safer and quicker coating process, using diclofenac-containing pellets prepared by extrusion spheronization as the core material (20 % drug content), and pure stearic acid (SA) and palmitic acid (PA) as the enteric hot-melt coating materials ("Development and evaluation of a hot-melt coating technique for enteric coating"; Brazilian Journal of Pharmaceutical Sciences; Vol. 48, N. 1, Jan./Mar., 2012). In a specially modified coating pan, coatings of molten stearic acid or palmitic acid were applied to the pellets to achieve coating levels of 5-15 % w/w. Patil et al. claim that hot-melt coating (HMC) with pure stearic acid or pure palmitic acid both exhibit very good enteric coating properties, with the coated pellets showing negligible drug release in acidic pH 1.2 and gradually increasing release at pH 6.8.

However, as observed by the inventors, Patil's approach of applying pure solid fatty acids, such as stearic acid and palmitic acid as a hot-melt coating did not prove to be successful when attempting the same coating applied to cores with a higher drug content and/or cores with more irregular shapes than Patil's spheronized pellets, such as drug crystals in the form of platelets or needles. Also other authors have challenged Patil's teachings on fatty acid coatings, stating that unlike prior art enteric polymers (which are often purposefully designed and carefully engineered to dissolve quickly enough upon contact with the media of increasing pH in the intestines), wax- and/or lipid-coatings are more suited for an extended and/or delayed drug release, and typically require lipolysis and digestion of the wax/lipid to allow said release (see e.g., Habashy et al. "An innovative wax-based enteric coating for pharmaceutical and nutraceutical oral products"; International Journal of Pharmaceutics 591 (2020) 119935).

For this reason, Habashy et al. suggested a wax/alginate-based enteric coating wherein e.g., theophylline containing tablets (drug content 50 %) were coated in a fluidized bed with heated O/W-emulsions comprising emulsified wax in aqueous alginate solutions. Waxes such as beeswax, cetyl palmitate, carnauba wax, or rice bran wax were tested but none proved as efficient of an enteric coating as a ceresin wax/alginate combination.

For said combination, coating levels of 10 % proved sufficient to prevent the tablets from uptaking gastric medium and to provide release profiles complying to pharmacopeial enteric release criteria. Like Patil et al., no tests were performed by Habashy et al., though, for cores with a higher drug content and/or cores with irregular shapes, such as drug crystals in the form of platelets or needles. Furthermore, applying multiple-phase systems such as O/W-emulsions is usually a more complex process and requires longer process times (e.g., due to drying times) than working with single-phase systems such as molten coating compositions. Wax/alginate-based enteric coatings may also be more challenging, and potentially not suitable, for acid-sensitive drugs and/or for highly watersoluble drugs (e.g., aqueous solubility of about ≤ 35 mg/mL at a temperature of 15-25 °C) since the alginate absorbs a high amount of acidic media into the coating.

It is thus an object of the present invention to provide hot-melt coated pH-dependent release particle(s), including particle(s) based on cores that a high active ingredient content (>50 %) and/or an irregular shape (such as drug crystals in the form of platelets or needles), and with said particle(s) being coated with improved hot-melt coatings that allow for pH-dependent release, preferably enteric release, from particle(s) coated therewith. A further object of the present invention to provide pharmaceutical or nutraceutical compositions comprising the thus coated particle(s). A yet further object of the present invention to provide a method for the preparation of the hot-melt coated pH-dependent release particle. Further objects of the invention will be clear on the basis of the following description of the invention, examples, and claims.

### SUMMARY OF THE INVENTION

In a first aspect, the invention relates to a hot-melt coated pH-dependent-release particle comprising a core and a coating, wherein the core comprises, or consists of, an active ingredient, and wherein the coating comprises, or consists of, a fatty acid which is solid at room temperature, and a CITREM ester; and wherein the pH-dependent release refers to a dissolution profile - as determined in an aqueous medium at 37 °C using a USP Dissolution Apparatus type 2 (paddle apparatus) at a stirring speed of 100 rpm - in which:
- during the initial acid stage in 750 mL 0.1 N hydrochloric acid, no more than 25 % of the active ingredient contained in the particle are released within 2 h, and
- during the buffer stage in 1000 mL pH 6.8 phosphate buffer, at least 75 % of the active ingredient contained in the particle is released within no more than 2 h in said pH 6.8 phosphate buffer.

In a second aspect, the present invention provides a pharmaceutical or nutraceutical composition comprising the particle according to the first aspect of the invention, or a plurality thereof.

In a third aspect, the present invention provides a method for the preparation of the hot-melt coated pH-dependent release particle according to the first aspect of the invention, comprising the steps of:
(a) providing a core particle comprising, or consisting of, an active ingredient,
(b) providing a coating composition that comprises, or consists of, a fatty acid which is solid at room temperature, and a CITREM ester,
(c) melting the coating composition,
(d) coating the core particle with the molten coating composition of step (c),
(e) cooling the product of step (d) to allow the molten composition to solidify and form the hot-melt coated pH-dependent release particle.

### DESCRIPTION OF THE FIGURES

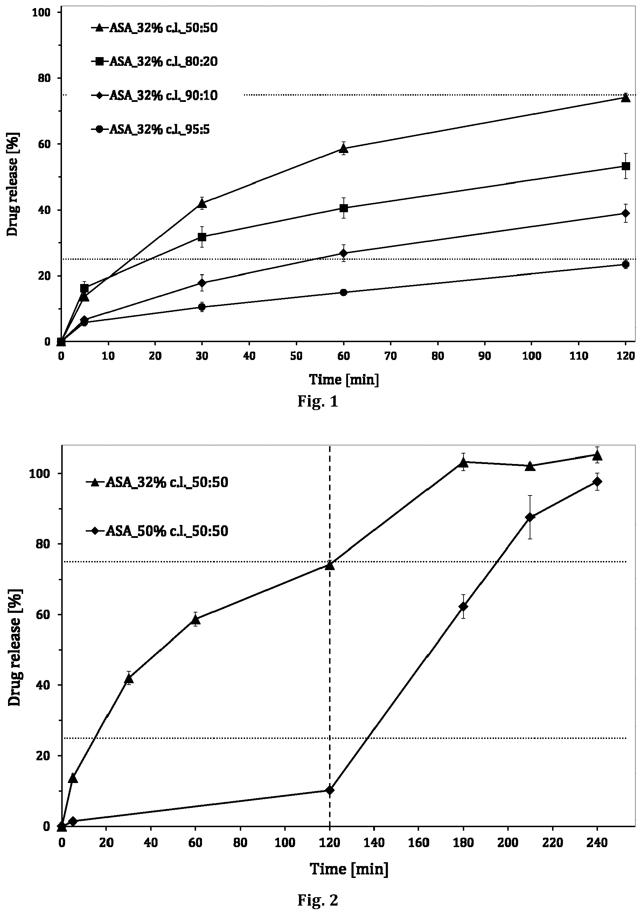

**Fig. 1** shows the acid-stage drug release (0.1 N HCl for 2) of crystalline acetylsalicylic acid (ASA) cores coated to 32 % coating level (based on the final weight of the pH-dependent release particle) according to the process described in Example 1 with coatings comprising mixtures of palmitic acid (PA) and CITREM ester (here 'BCFS') in different PA/BCFS weight ratios.
**Fig. 2** shows the drug release of acetylsalicylic acid (ASA) cores coated to different coating levels (32 and 50 % based on the final weight of the pH-dependent release particle) according to the process described in Example 1 with a 50:50 PA/BCFS coating; release in 0.1 N HCl for the first 2 h, thereafter in pH 6.8 phosphate buffer with 0.2 vol.-% polysorbate addition (here Tween^{®} 80).

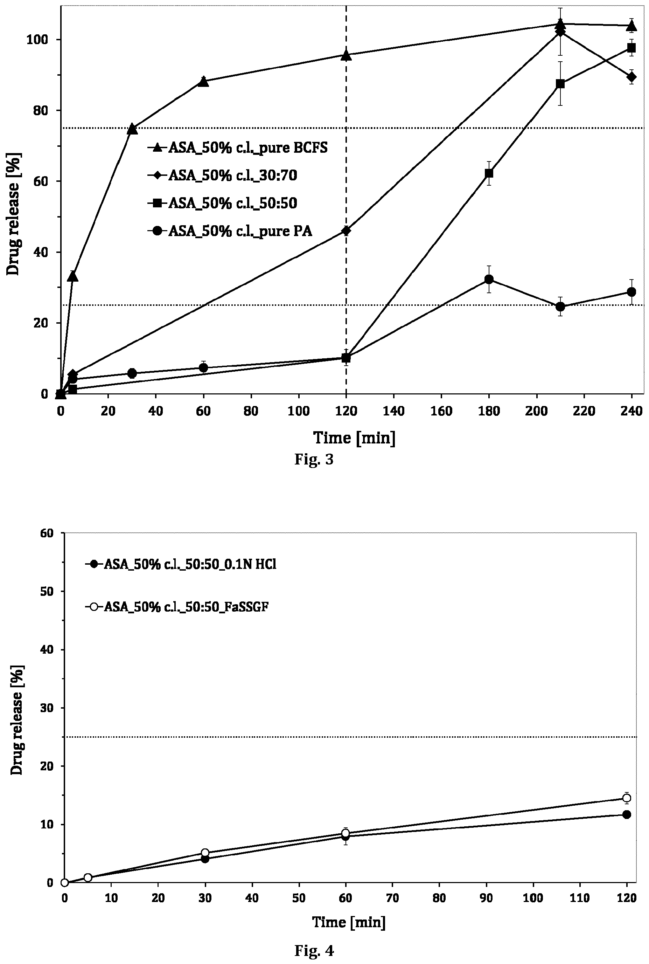

**Fig. 3** shows the drug release of crystalline acetylsalicylic acid (ASA) cores coated to 50 % coating level (based on the final weight of the pH-dependent release particle) according to the process described in Example 1 with either pure palmitic acid (PA), pure CITREM ester (here 'BCFS'), and coatings comprising PA/BCFS-mixtures in different ratios; release in 0.1 N HCl for the first 2 h, thereafter in pH 6.8 phosphate buffer with 0.2 vol.-% polysorbate addition (here Tween^{®} 80).
**Fig. 4** shows the drug release of crystalline acetylsalicylic acid (ASA) cores coated to 50 % coating level (based on the final weight of the pH-dependent release particle) according to the process described in Example 1 with PA/BCFS 50:50-coating; release in 0.1 N HCl compared to release in a fasted-state simulated gastric fluid release medium (FaSSGF).

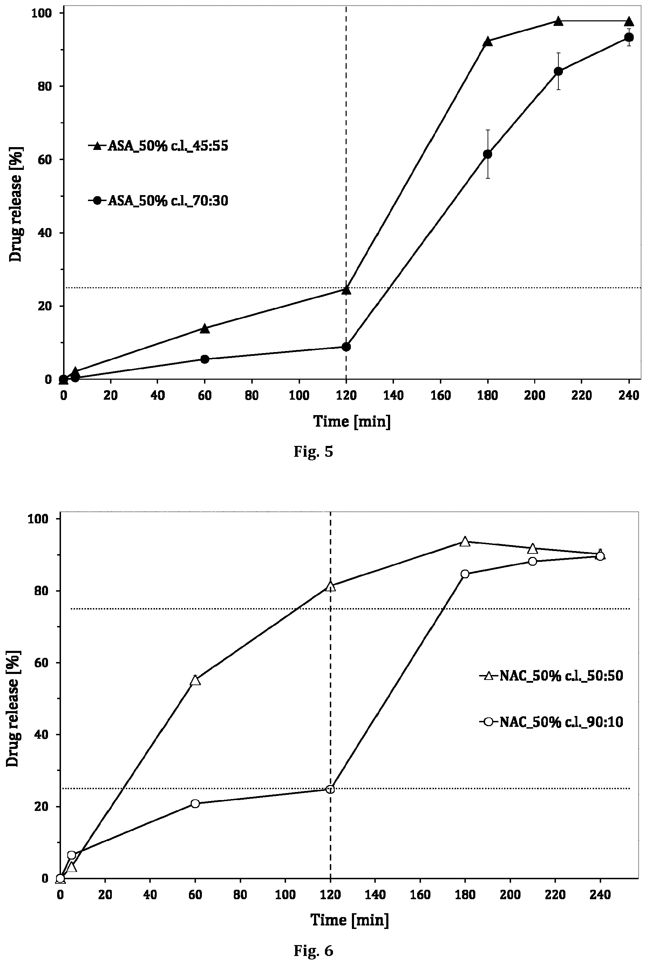

**Fig. 5** shows the drug release of crystalline acetylsalicylic acid (ASA) cores coated to 50 % coating level (based on the final weight of the pH-dependent release particle) according to the process described in Example 2 with PA/BCFS-coatings in different weight ratios; release in 0.1 N HCl for the first 2 h, thereafter in pH 6.8 phosphate buffer with 0.2 vol.-% polysorbate addition (here Tween^{®} 80).
**Fig. 6** shows the drug release of crystalline N-acetylcysteine (NAC) cores coated to 50 % coating level (based on the final weight of the pH-dependent release particle) according to the process described in Example 2 with PA/BCFS-coatings in different weight ratios; release in 0.1 N HCl for the first 2 h, thereafter in pH 6.8 phosphate buffer with 0.2 vol.-% polysorbate addition (here Tween^{®} 80).

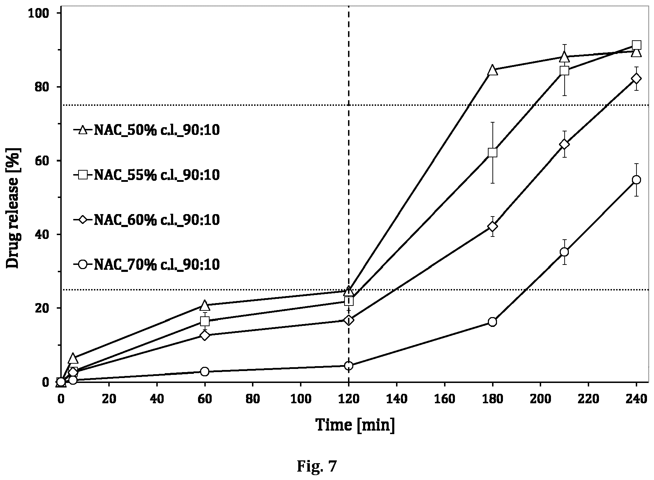

**Fig. 7** shows the drug release of crystalline N-acetylcysteine (NAC) cores coated according to the process described in Example 2 with a 90:10 PA/BCFS-coatingto different coating levels (50, 55, 60, and 70 % based on the final weight of the pH-dependent release particle); release in 0.1 N HCl for the first 2 h, thereafter in pH 6.8 phosphate buffer with 0.2 vol.-% polysorbate addition (here Tween^{®} 80).

### DETAILED DESCRIPTION OF THE INVENTION

In a first aspect, the invention relates to a hot-melt coated pH-dependent-release particle comprising a core and a coating, wherein the core comprises, or consists of, an active ingredient, and wherein the coating comprises, or consists of, a fatty acid which is solid at room temperature, and a CITREM ester; and wherein the pH-dependent release refers to a dissolution profile - as determined in an aqueous medium at 37 °C using a USP Dissolution Apparatus type 2 (paddle apparatus) at a stirring speed of 100 rpm - in which:
- during the initial acid stage in 750 mL 0.1 N hydrochloric acid, no more than 25 % of the active ingredient contained in the particle are released within 2 h, and
- during the buffer stage in 1000 mL pH 6.8 phosphate buffer, at least 75 % of the active ingredient contained in the particle is released within no more than 2 h in said pH 6.8 phosphate buffer.

Within the scope of the present invention, the term 'pH-dependent release' thus refers to a release of the active ingredient that is slow in acidic media (specifically, no more than 25 % within 2 h in 0.1 N HCl) and faster in basic media (specifically, at least 75 % within 2 h after the shift from 0.1 N HCl to pH 6.8 phosphate buffer). The term 'pH-dependent release' thus encompasses, or includes, more narrow forms thereof, such as the so-called 'enteric release' (as defined further below), but is not meant to be identical or synonymous therewith.

It should further be understood that terms such as 'active ingredient', 'drug', or 'active agent' are used synonymously herein, and refer to the active compound provided inside the core of the coated particle of the first aspect of the invention for the purpose of its pH-dependent release. Most commonly, said active compound may be of either nutraceutical or pharmaceutical value to the subject ingesting the coated particle(s) (such as an iron supplement or a painkiller); in some cases both.

Also, as used herein, all percentages, parts, and/or ratios are intended to be by weight of the total weight; i.e., '%' should be read as 'wt.-%', unless otherwise specified, or indicated, or required by the context.

The term 'room temperature', as used herein, shall be understood as 20 ± 5 °C, or in other words as ranging from 15 °C to 25 °C, as defined, for instance, by the European Pharmacopoeia or by the WHO guidance 'Guidelines for the Storage of Essential Medicines and Other Health Commodities' (2003).

The term 'CITREM ester', as used herein, refers to citric acid esters of mono- and diglycerides of fatty acids, specifically of edible fatty acids; often, mono- and diglycerides based on either sunflower oil and/or palm oil. Sometimes, these esters are also referred to as citroglycerides, citric acid glyceride esters, or monoglyceride citrate. CITREM esters are hydrophilic O/W-emulsifiers with a hydrophilic-lipophilic balance (HLB) in the range of about 8-12 which are commonly used in food products such as ice cream, sausages, margarine, whipped creams, or infant formulae, where they are usually listed with their European food number, or 'E-number', E472c (as opposed to, for instance, other esters such as LACTEM, E472b, the lactic acid ester of mono- and diglycerides).

The shift from the initial acid stage to the subsequent buffer stage - mimicking the transition from the stomach into the intestines - may be obtained by either (i) physically transferring the particle(s) from the 0.1 N hydrochloric acid as the acidic aqueous medium into a basic, preferably prewarmed, aqueous medium exhibiting a pH of 6.8; or by (ii) adding a concentrated phosphate buffer solution to the 0.1 N hydrochloric acid in such an amount that the resulting phosphate buffer, as the basic aqueous medium, exhibits a pH of 6.8 (such as adding 250 mL of a trisodium phosphate dodecahydrate buffer solution to 750 mL of the 0.1 N HCl). The first approach is more commonly used when working with a USP Dissolution Apparatus type 1 (rotating basket apparatus) since it 'traps' the particle(s) within the rotating baskets and hence allows for an easy and fast physical transfer thereof into another medium. The latter technique is more commonly used when working with a paddle-stirrer type of dissolution tester; it is also referred to as Method A in the European Pharmacopoeia monography 2.9.3. (Ph. Eur. 10th edition). With respect to said pH-change of the aqueous medium, expressions such as "within no more than 2 h in said pH 6.8 phosphate buffer" are referring to times in addition to the initial 2 h in the acidic medium.

The coated particles according to the first aspect of the invention, or, in other words, the approach of combining fatty acids which are solid at room temperature (e.g., palmitic acid, or stearic acid), with a CITREM ester in a hot-melt coating composition, differs from prior art teachings such as Patil et al., which allegedly achieve enteric release, or any form of pH-dependent release, by applying pure stearic acid or palmitic acid coatings only.

In that regard, reference is made, for instance, to **Fig. 3**, which inter alia shows the drug release of crystalline acetylsalicylic acid (ASA) cores coated - according to the process described below in Example 1 - with pure palmitic acid (PA) to a coating level of 50 % based on the final weight of the pH-dependent release particle As can be seen in this figure, pure palmitic acid alone was not at all effective in achieving pH-dependent release. While after the first two hours in 0.1 N HCl (the acid stage), only about 10 % of the drug contained in the coated ASA-particles was released, the subsequent release during the buffer stage in a pH 6.8 phosphate buffer with 0.2 vol.-% polysorbate addition (here Tween^{®} 80) did not increase significantly and achieved only about 30 % after another two hours (i.e., 4 h in total).

This outcome may, in parts, be the result of the thick coating applied. Yet, when choosing a lower coating level, the drug release during the acid stage is often not low enough; for instance, reaching already about 42 % within the first two hours in 0.1 N HCl for a coating level of 32 % (graph not shown). In light of the already too fast ASA-release observed at 32 % coating level, lower coating levels of pure palmitic acid, such as those described by Patil et al. (5 to 15 %), have not been tested anymore for this drug.

Without wishing to be bound by theory, it is speculated by the inventors that due to their oblong, needle-like shape, the ASA-crystals used as the core material for the coated ASA-particles require a thicker coating to prevent excessive premature release during the acid stage than the far more regular, smooth, round diclofenac-containing pellets used as cores by Patil et al. (Patil had formulated their diclofenac salt into spherical pellets with only 20 % drug content, using microcrystalline cellulose, lactose monohydrate, and povidone as excipients).

As can further be seen from **Fig. 3**, a comparative coating with the CITREM ester alone (here the 'BCFS' ester, as described in more detail further below) to the same coating level of 50 % based on the final weight of the coated particle was not sufficient to slow down drug release in the first two hours in 0.1 N HCl, with about 80 % of the drug ASA being released already after 2 h despite the thick coating.

The inventors found that combining lipophilic fatty acids which are solid at room temperature (such as the palmitic acid or stearic acid suggested by Patil et al.), with the more hydrophilic CITREM esters in hot-melt coating compositions with different weight ratios of these two components can be employed as a means to control the release from drug particles coated therewith in both the acid stage and the buffer stage (i.e., achieving pH-dependent release). Furthermore, this can be achieved even for drug cores which exhibit less regular shapes, such as drug crystals in the form of needles or platelets. Thereby the invention does not only provide an adjustable means to achieve pH-dependent drug release but also offers improved time- and energy-efficiency since the drug does not need to be formulated into a spherical, or spheroidal, core in a separate step first.

In one embodiment, the pH-dependent release refers to a dissolution profile (determined as described above) in which during the initial acid stage in 750 mL 0.1 N hydrochloric acid, no more than 20 % of the active ingredient contained in the particle are released within 2 h, and during the buffer stage in 1000 mL pH 6.8 phosphate buffer, at least 75 % of the active ingredient contained in the particle is released within no more than 1.5 h in said pH 6.8 phosphate buffer.

In a specific embodiment, the pH-dependent release refers to a dissolution profile (determined as described above) in which during the initial acid stage in 0.1 N hydrochloric acid, no more than 15 % of the active ingredient contained in the particle are released within 2 h, and during the buffer stage in 1000 mL pH 6.8 phosphate buffer, at least 75 % of the active ingredient contained in the particle is released within no more than 1 h in said pH 6.8 phosphate buffer.

In a more specific embodiment, the pH-dependent release refers to a dissolution profile (determined as described above) in which during the initial acid stage in 0.1 N hydrochloric acid, less than 10 % of the active ingredient contained in the particle are released within 2 h, and during the buffer stage in 1000 mL pH 6.8 phosphate buffer, at least 75 %, preferably at least 80 %, and further preferably at least 85 %, of the active ingredient contained in the particle is released within no more than 0.75 h in said pH 6.8 phosphate buffer. This embodiment is also reflected in the enteric release requirements of most common pharmacopoeias such as the European pharmacopoeia (e.g., Ph. Eur. 10^{th} ed.; 2.9.3.), or the U. S. pharmacopoeia (USP), and is one of the preferred embodiments of pH-dependent release, in particular, for acid-sensitive drugs.

Based on the specific pharmacopoeial monography concerned, the time in the buffer stage that is still considered suitable for enteric release may sometimes be longer than 0.75 h; for instance, the US-pharmacopoeia allows for up to 1.5 h for at least 75 % release of acetylsalicylic acid (ASA) to be reached. Thus, in yet more specific embodiments, the pH-dependent release can refer to a dissolution profile (determined as described above) in which during the initial acid stage in 0.1 N hydrochloric acid, less than 10 % of the active ingredient contained in the particle are released within 2 h, and during the buffer stage in 1000 mL pH 6.8 phosphate buffer, at least 75 % of the active ingredient contained in the particle is released within no more than 1.5 h, preferably within no more than 1 h, in said pH 6.8 phosphate buffer.

The term 'acid-sensitive drugs' as used herein refers to, and includes, any drugs whose pharmaceutical or nutraceutical efficacy is reduced and/or otherwise negatively affected when coming into contact with the acidic media of the stomach, including both (i) drugs which are destroyed and/or deactivated by said acidic media, but also (ii) drugs which are sensitive to premature activation and/or poor absorbability if coming into contact with said acidic media in the inner lumen of the stomach rather than - as intended - within the stomach's parietal cells (e.g., proton-pump inhibitors).

As mentioned above, it should be understood that within the scope of the present invention, and as reflected by claim 1, the term 'pH-dependent release' is not meant to be identical or synonymous with the term 'enteric release'. Instead, the term 'pH-dependent release' is broader; mainly, in that it allows higher amounts of up to 25 % of the drug contained in the coated particle(s) to be released in the initial acidic stage already. While the more narrow 'enteric release' provisions are favored and advisable, especially for acid-sensitive drugs, the above-mentioned broader forms of pH-dependent release achieved by the particle (s) according to the first aspect of the invention, are suited, for instance, for drugs with a narrow absorption window in the upper intestine, or for gastro-irritant drugs which would aggravate the gastric mucosa in case the full dose is brought in contact with said mucosa within a very short time-frame (such as when taking an immediate-release formulation of a non-steroidal anti-inflammatory drug, or NSAID, for instance).

In one embodiment, a surfactant is added to at least the pH 6.8 phosphate buffer during the buffer stage; optionally to both the acidic and the basic aqueous release media. This approach can be chosen, for instance, as a means to mimic the surface tension of biorelevant media such as fasted-state simulated intestinal fluid (FaSSIF). Optionally, the surfactant is added to at least the pH 6.8 phosphate buffer during the buffer stage at a concentration of 0.01 to 0.5 vol.-% based on the volume of the pH 6.8 phosphate buffer. In a specific embodiment, the surfactant is a polysorbate, optionally polysorbate 80 (e.g.,. In one embodiment, 0.2 vol.-% of polysorbate 80 is added, during the buffer stage, to the 1000 mL of pH 6.8 phosphate buffer. These surfactant additions, while not mandatory, were considered useful by the inventors nonetheless to be as close as possible to biorelevant media such as fasted-state simulated intestinal fluid (FaSSIF);
for comparison, FaSSIF exhibits a surface tension of about 51 mN/m whereas the phosphate-buffered release medium in the buffer stage with 0.1 to 0.2 vol.-% Tween^{®} 80 addition exhibit surface tensions of about 46 to 49 mN/m.

Similar polysorbate 80 additions to the 0.1 N HCl medium during the acid stage are typically not useful since this surfactant degrades in 0.1 N HCl. And while a more acid-stable surfactant may theoretically be employed in the 0.1 N HCl medium during the acid stage to mimic the surface tension of biorelevant media, this was not considered necessary for the purpose of the present invention. In this regard, reference is made to **Fig. 4**, which shows the drug release of crystalline acetylsalicylic acid (ASA) cores coated - according to the process described below in Example 1 - to a coating level of 50 % based on the final weight of the coated particle with a PA/BCFS 50:50-coating in either 0.1 N HCl or a fasted-state simulated gastric fluid release medium (FaSSGF).

As can be seen in this figure, the release of ASA in 0.1 N HCl over the first two hours shows no, or at best very minor, differences compared to the release in FaSSGF. This is, at least in parts, explainable by the similar surface tensions of the two release media: about 69 mN/m for 0.1 N HCl versus 65 mN/m for FaSSGF.

In one embodiment, the coating comprises at least 2 % of the CITREM ester, preferably at least 3 %, more preferably at least 5 %. As mentioned above, working with pure fatty acids such as palmitic or stearic acid, as Patil et al. suggested, turned out unsuccessful, at least when working with drug cores that were not pre-formulated into a spherical, or spheroidal, starter core prior to the hot-melt coating. The release was extended by applying, for instance, pure palmitic acid, but little to no pH-dependency was found. Upon addition of CITREM esters to the hot-melt coating, however, pH-dependency could be achieved and tailored via the content of the CITREM ester, and, more specifically, its ratio to the fatty acid.

In one embodiment, the weight ratio of the fatty acid and the CITREM ester is in the range of 45:55 to 98:2, or 50:50 to 97:3, or 60:40 to 95:5, or 70:30 to 90:10.

In one embodiment, the fatty acid is a C₁₃-C₂₂ fatty acid, a saturated fatty acid, or a saturated C₁₃-C₂₂ fatty acid. In a further embodiment, the fatty acid has a melting point in the range of 55-75 °C, or from 60-70 °C, such as 62 °C, or 63 °C, or 69 °C. In a yet further embodiment, the fatty acid is palmitic acid and/or stearic acid. In one of the preferred embodiments, the fatty acid is palmitic acid.

In one embodiment, the CITREM ester is a partially neutralized citric acid ester of mono- and diglycerides (E472c) combined with mono- and diglycerides (E471); for reasons of brevity and conciseness herein referred to as BCFS or BCFS-ester. An exemplary and commercially available BCFS-ester that is suitable for the present invention is the Grindsted^{®} CITREM ester called GRINDSTED^{®} CITREM BC-FS SG, which exhibits a melting point of about 63 °C, a melt viscosity of 73.69 ± 1.05 mPas at 100 °C, an HLB-value of about 11, an acid value of 20-35, a saponification value of 225-255, as well as the following typical fatty acid composition; C₁₄ < 3 %, C₁₆ 38-60 %, C₁₈ 38-58 % and C₂₀ < 2 %.

The inventors surprisingly found that the BCFS-ester is beneficial over other commercially available CITREM esters such as GRINDSTED^{®} CITREM N12 Veg SG (hereinafter referred to as 'N12' only) in that it does not start to smell unpleasantly during the storage. GRINDSTED^{®} CITREM N12 Veg SG is a partially neutralized citric acid ester of mono- and diglycerides (E472c) exhibiting a melting point of about 64 °C, a melt viscosity of 213.06 ± 2.47 mPas at 100 °C, an HLB-value of about 12, an acid value of 10-25, a saponification value of 220-250, an iodine value of max. 3, a citric acid content of min. 13 %, as well as the typical fatty acid composition C₁₄ 1 %, C₁₆ 44 %, C₁₈ 54 %, and C₂₀ < 1 %. It lacks the BCFS's addition of the mono- and diglycerides (E471). With the N12-ester, similar release results in terms of pH-dependent release were achievable as with BCFS; however, in light of the smell-formation during storage, that was more pronounced with N12, BCFS-esters are favored for the present invention. The inventors also noticed that the viscosity of PA/N12-melt compositions at a given temperature was higher than that of comparable PA/BCFS-melt compositions, rendering the PA/N12-melt compositions slightly less readily manageable than PA/BCFS. Also for this reason, BCFS-esters are favored for the present invention.

One of the advantages of combining palmitic acid with CITREM ester such as BCFS or N12 is that they exhibit very similar melting points (palmitic acid ∼62 °C; BCFS ∼63 °C; N12 ∼64 °C) and recrystallization behaviors, and are thus less likely to separate from one another during processing, e.g., when an applied melt-coating is cooling down.

In one embodiment, the coating is substantially free from water-swellable polymers, in particular, polysaccharides, such as cellulosic polymers (e.g., cellulose ethers), alginic acid derivatives (e.g., alginates), or polyacrylate derivatives. The approach of adding water-swellable polymers to a coating, such as the fatty acid coating described herein, for instance as pore-formers, has been chosen in some of the prior art processes and products attempting a lipid-based pH-dependent coating. However, this approach is considered less beneficial for pH-dependent release than the CITREM ester addition of the present invention, especially for acid-sensitive drugs, because even if the drug release in an acidic medium may be low (preferably < 10 % during the first two hours), said acidic medium may still get inside the coated particle via the 'swollen-polymer pores' and thus get in contact with the acid-sensitive drug.

In one embodiment, the coating essentially consists of the fatty acid and the CITREM ester. For instance, in a specific embodiment, the coating essentially consists of palmitic acid and, as the CITREM ester, a partially neutralized citric acid ester of mono- and diglycerides (E472c) combined with mono- and diglycerides (E471); or in other words, the coating consists of palmitic acid and the above mentioned BCFS-ester, such as the commercially available Grindsted^{®} CITREM ester GRINDSTED^{®} CITREM BC-FS SG. In a more specific embodiment, the coating essentially consists of palmitic acid and, as the CITREM ester, a partially neutralized citric acid ester of mono- and diglycerides (E472c) combined with mono- and diglycerides (E471), i.e., the above mentioned 'BCFS' ester (such as GRINDSTED^{®} CITREM BC-FS SG); and the coating comprises at least 2 % of this CITREM ester, preferably at least 3 %, more preferably at least 5 %.

In a further embodiment, the coating essentially consists of palmitic acid and, as the CITREM ester, a partially neutralized citric acid ester of mono- and diglycerides (E472c) combined with mono- and diglycerides (E471); and the weight ratio of the fatty acid and this CITREM ester is in the range of 45:55 to 98:2, or 50:50 to 97:3, or 60:40 to 95:5, or 70:30 to 90:10.

With respect to these weight ratios of the fatty acid and the CITREM ester (e.g., palmitic acid and the BCFS-ester), reference is made, for instance, to **Fig. 3** and **Fig. 5**, which show the drug release of crystalline acetylsalicylic acid (ASA) cores coated - according to the processes described below in Examples 1 and 2 for **Fig. 3** and **Fig. 5**, respectively - to a coating level of 50 % based on the final weight of the pH-dependent release particle with either pure palmitic acid (PA), pure CITREM ester (here 'BCFS'), and coatings comprising PA/BCFS-mixtures in different weight ratios (30:70, 45:55, 50:50 and 70:30). As can be seen in **Fig. 3**, neither hot-melt coatings ofpure CITREM ester (here, BCFS) nor those comprising too little of the release-retarding fatty acid (here palmitic acid) succeeded in sufficiently reducing the drug release during the initial acid stage of two hours to no more than 25 %. For instance, although clearly being slower than the pure BCFS-coating alone, the batch coated with a 30:70 PA/BCFS blend still released almost half of the drug contained in the coated particles during the initial acid stage.

Reference is also made to **Fig. 6**, which shows the drug release of crystalline N-acetylcysteine (NAC) cores coated - according to the processes described below in Example 2 - to a coating level of 50 % based on the final weight of the pH-dependent release particle with coatings comprising PA/BCFS-mixtures in different weight ratios (50:50 and 90:10) Unlike the gastro-irritant drug acetylsalicylic acid (ASA), N-acetylcysteine (NAC) does not typically require a pH-dependent release with no more than 25 % of the drug being released during the initial 2 h acid stage, let alone an enteric release with less than 10 % drug release; NAC was predominantly chosen as a crystalline model drug with a pH-independent and higher solubility than ASA (ASA: 2.24 g/L; NAC: 200 g/L). As can be seen in **Fig. 6**, the batch coated with a 50:50 PA/BCFS blend released about 80 % of the drug contained in the coated particles during the initial acid stage already, which is believed to be the result of NAC's higher solubility compared to ASA. However, with the 90:10-blend, the release of NAC during the initial acid stage could be reduced to no more than 25 % while allowing for at least 75 % to be released in the next two hours during the subsequent buffer stage (here about 85 % already within the first hour in the pH 6.8 phosphate buffer). In other words, within the weight ranges provided above (e.g., 45:55 to 98:2), it is well possible to adjust the pH-dependent release based on the solubility of the drug to be released by choosing coatings with higher fatty acid contents and/or lower CITREM ester contents for drugs with higher solubility, and vice versa.

In on embodiment, the coating level, based on the final weight of the pH-dependent release particle, is 25 % or higher, or 30 % or higher, or 32 % or higher, 35 % or higher. For instance, in one embodiment, the coating level, based on the final weight of the pH-dependent release particle, is in the range of 25-70 %, or in the range of 30-70 %, or in the range of 30-65 %, or in the range of 35-60 %. In specific embodiments, the coating level, based on the final weight of the pH-dependent release particle, is 32 %, 40 %, 50 %, 55 %, or 60 %. Lower or higher coating levels would be possible in theory; for instance, for drugs with a solubility lower than that of acetylsalicylic acid (ASA) or higher than that of N-acetylcysteine (NAC), respectively. Care should be taken, though, especially on the end of lower coating levels, and especially for irregularly shaped drug particles such as crystal needles or the like, that the complete surface of said drug particles is covered nonetheless with the hot-melt coating.

With respect to the coating levels of the fatty acid and the CITREM ester compositions (e.g., palmitic acid and the BCFS-ester), reference is made, for instance, to **Fig. 1**, **2**, and **7**. **Fig. 1** shows the acid-stage drug release (0.1 N HCl for 2) of crystalline acetylsalicylic acid (ASA) cores - coated according to the process described below in Example 1 - to a coating level of 32 % based on the final weight of the coated particle with coatings comprising mixtures of palmitic acid (PA) and CITREM ester (here 'BCFS') in different PA/BCFS weight ratios, namely, 50:50, 80:20, 90:10 and 95:5. As can be seen in **Fig. 1**, the ASA-release during the acid stage in 0.1 N HCl could be gradually reduced from about 75 % at 2 h with the 50:50 PA/BCFS ratio to about 23 % at 2 h with the 95:5 PA/BCFS ratio. However, in case one of the more permeable coating compositions, such as 50:50, is favoured, pH-dependent release within the requirements of claim 1 is still feasible upon increasing the coating level from 32 % to 50 %, as is shown for ASA in **Fig. 2****.** Vice versa, for a drug with a solubility lower than ASA, also coating levels lower than 32 %, such as 25-31 %, may be suitable.

Similarly, **Fig. 7** depicts the impact of the coating level on the drug release of crystalline N-acetylcysteine (NAC) cores coated - according to the process described below in Example 2 - with a 90:10 PA/BCFS coating to different coating levels, namely, 50, 55, 60 and 70 % based on the final weight of the coated particle. All depicted coating levels achieve the desired slow release during the acid stage in 0.1 N HCl (i.e., not more than 25 % in 2 h), but only 50 to 60 % allow for sufficiently fast release in the subsequent buffer stage in the pH 6.8 phosphate buffer (i.e., at least 75 % within the next 2 h). At the highest coating level (here 70 %), the release during the buffer stage is increased compared to the release in 0.1 N HCl but overall still too slow, reaching only about 55 % NAC-release after 2 h in the pH 6.8 phosphate buffer. For a drug with solubility higher than NAC, though, even coating levels of about 70 % or higher may be suitable.

In summary, by combining fatty acids with CITREM esters in hot-melt coating compositions as described herein, the release from drug particles coated therewith according to the first aspect of the invention can be controlled in both the acid stage and the buffer stage (i.e., achieving pH-dependent release), and can be tailored to the requirements of specific drugs by selecting suitable weight ratios of the fatty acid and CITREM ester as well as suitable coating levels within the above-described parameter ranges (e.g., PA/BCFS 45:55 to 98:2 at coating levels between 25-70 %).

The pH-dependent release particle according to the first aspect of the invention can be used for a variety of drugs, exhibiting low or high solubilities, and/or pH-dependent or pH-independent solubilities, as represented herein by the model-drugs acetylsalicylic acid (ASA) or N-acetylcysteine (NAC), respectively.

In one embodiment, the active ingredient is selected from the group consisting of acid-sensitive drugs, drugs sensitive to enzymatic degradation, gastro-irritant drugs, poorly tasting drugs, and/or drugs with an absorption window in the small intestine.
In a specific embodiment, the active ingredient is selected from the group consisting of: proton-pump inhibitors, analgesics such as non-steroidal anti-inflammatory drugs (NSAIDs), bisphosphonates, colitis drugs, fumaric acid compounds, glucocorticoids, antidepressants, expectorants, enzymes, vitamins, mineral salts, plant extracts, omega-3 fatty acids, antioxidants, and probiotics. In a more specific embodiment, the active ingredient is a non-steroidal anti-inflammatory drug (NSAID), e.g., acetylsalicylic acid.
In an alternative embodiment, the active ingredient is an expectorant, e.g., N-acetylcysteine.

In one embodiment, the active ingredient exhibits a median particle size (D50) in the range of 100 µm to 300 µm, or 125 µm to 275 µm, or 150 µm to 250 µm, as measured by dynamic image analysis, e.g., using a Camsizer^{®} XT device. Any particle size provisions as used herein - either measured or calculated/derived from measured values - refer to values as obtained by dynamic image analysis (e.g., according to ISO 13322-2) and unless mentioned otherwise to values obtained using a Camsizer^{®} XT device (Retsch Technology GmbH, Haan, Germany) equipped with the X-Jet plug-in cartridge and its related software. The Camsizer^{®} set-up employs a dynamic imaging technique in which particle samples are dispersed by pressurized air, passed through a gap illuminated by pulsed LED-light sources, and their images (more specifically their projections) then recorded by two digital cameras and analyzed for size and shape in order to determine a variety of length and width descriptors for the particles, including weighted arithmetic mean particle sizes as well as D10, D50 and D90-values. The Camsizer^{®} XT device is preferred for particle size determinations according to the invention since it allows for the precise and reproducible analysis of particle sizes of fine powders down to 1 µm. This, however, should not be misconstrued in such a way as to exclude, or prohibit, the use of laser diffraction or any other established methods for particle size determinations in the required micrometer-range. In cases where the particle sizes determined with other techniques differ from those obtained with the Camsizer^{®} set-up, the results obtained with the Camsizer^{®} shall prevail.

In one embodiment, the core of the coated particle contains more than 50 %, or more than 60 %, or more than 70 %, or more than 80 %, or more than 90 %, or more than 95 % of the active ingredient, based on the weight of the core. In a specific embodiment, the core of the coated particle consists of the active ingredient; or, in other words, the coating is applied directly to the drug raw material. The approach is beneficial in that it obviates the need to first formulate the raw drug powder (e.g., crystals of the drug) into a drug-containing starter core prior to the actual coating step. In some cases, this additional step does not only require added time, excipients, and/or energy but also a different device. As mentioned above, care should be taken, though, especially for irregularly shaped drug particles such as crystal needles, platelets, or the like, that the complete surface of said drug particles is covered nonetheless with the hot-melt coating. This may, in some cases, require higher coating levels than, for instance, with drug-containing cores which were first formulated into smooth, spherical pellets.

In one embodiment, the particle according to the first aspect of the invention is storage stable, i.e., the particle not changing its physicochemical properties during storage.
For instance, the coated particle does not start to stick to surfaces (either the insides of a storage container and/or to any neighbouring coated particles), and/or the coated particle does not start to develop any unpleasant smells during storage. In a specific embodiment, the particle's release profile is storage stable, i.e., the particle does not, or at the most minimally, change its release profile during storage.

In one embodiment, the particle according to the first aspect of the invention exhibits a median particle size (D50) in the range of 200 µm to 400 µm, or 225 µm to 375 µm, or 250 µm to 350 µm, as measured by dynamic image analysis, e.g., using a Camsizer^{®} XT device.

In one embodiment, the particle according to the first aspect of the invention is orally ingestible.

In a second aspect, the present invention provides a pharmaceutical or nutraceutical composition comprising the particle according to the first aspect of the invention, or a plurality thereof. Accordingly, any embodiments, or specific or preferred embodiments, disclosed herein in connection with the particle according to the first aspect of the invention, may be applied to the pharmaceutical or nutraceutical composition according to this second aspect of the invention.

In one embodiment, said composition is orally ingestible. For instance, in a specific embodiment, said composition is formulated as (i) granules, such as dispersible granules, effervescent granules, direct-to-mouth granules; or as (ii) tablets, such as dispersible tablets, effervescent tablets, or orally disintegrating tablets; or as (iii) granules filled into capsules.

In a third aspect, the present invention provides a method for the preparation of the hot-melt coated pH dependent release particle according to the first aspect of the invention, comprising the steps of:
(a) providing a core particle comprising, or consisting of, an active ingredient,
(b) providing a coating composition that comprises, or consists of, a fatty acid which is solid at room temperature, and a CITREM ester,
(c) melting the coating composition,
(d) coating the core particle with the molten coating composition of step (c),
(e) cooling the product of step (d) to allow the molten composition to solidify and form the hot-melt coated pH-dependent release particle.

Any embodiments, or specific or preferred embodiments, disclosed herein in connection with the particle according to the first aspect of the invention, may be applied to the preparation method according to this third aspect of the invention. For instance, the same coating materials and compositions thereof as described above for the particle according to the first aspect may be used in said preparation method. Same applies to features such as the coating levels as described above.

As mentioned above, coating with molten coating compositions is beneficial since they comprise the coating materials only; in other words, the application efficiency is very high, and no expensive removal of solvents (organic or aqueous) is needed. Furthermore, it is often easier and less time-consuming to prepare and adapt an optimized molten coating composition than, for instance, preparing an aqueous polymer dispersion.

In one embodiment, the molten coating composition of step (c) is applied in step (d) by pouring, dripping, and/or spraying it into, or onto, the core particles provided in step (a). In a specific embodiment, the core particles provided in step (a) are spray-coated with the molten coating composition of step (c). The temperature used for melting the coating composition should be chosen high enough to allow for the melting of the main coating composition components, namely, the fatty acid and the CITREM ester (preferably, of all coating composition components), yet low enough to prevent unnecessary energy expenditure and/or thermal stress on the drug cores throughout the coating process.

In one embodiment, the provided core particles of step (a) are coated, typically spray-coated, in a fluidized-bed coater. Fluidized-bed coaters are preferred in so far as they allow an even and quick application of the molten coating composition with a manageably low risk of the nascent hot-melt coated particles sticking together instead of being individually coated. Fluidized-bed coaters are also favored for their ability to handle cores of differently shaped core materials, such as oblong or needle-like shaped drug crystals versus spherical or spheroidal drug crystals.

In one embodiment, the temperature of the inlet air during step (d) - for instance, the inlet air flowing through a fluidized-bed coater - is in the range of 30 °C to 50 °C; or in the range of 35 °C to 48 °C; or in the range of 38 °C to 45 °C.

In one embodiment, the spray-rate of the molten coating composition during step (d) per kilogram of to-be-coated product - for instance, the spray-rate chosen for spraying the molten coating composition in(to) a fluidized-bed coater - is in the range of about 13 to 31 g/kg/min; or in the range of about 17 to 27 g/kg/min; or in the range of about 20 to 25 g/kg/min, such as about 22 g/kg/min.

In one embodiment, the process is carried out in a lab-scale fluidized-bed coater (e.g., a Ventilus^{®}V-2.5), and the spray-rate of the molten coating composition during step (d) is in the range of about 3.0 to 7.0 g/min; or in the range of about 4.0 to 6.0 g/min; or in the range of about 4.5 to 5.5 g/min, such as about 5.0 g/min.

The following examples serve to illustrate the invention; however, they should not be understood as restricting the scope of the invention.

### EXAMPLES

### Example 1 - Hotmelt-coating of acetylsalicylic acid (ASA) drug crystals

Acetylsalicylic acid (ASA) powder with a median particle size D50 of 176.5 µm, as determined by dynamic image analysis using a Camsizer^{®} XT device (Retsch Technology GmbH, Germany), was used as the core material. The drug material was used 'as is', i.e., no added steps were taken prior to the hot-melt coating step to formulate it into ASA-containing drug cores. In other words, the cores of the coated particles essentially consist of ASA. The ASA-drug crystals exhibit an oblong, needle-like shape.

Molten coating compositions consisting of either pure palmitic acid (PA; here, palmitic acid (≥ 98 %) by Carl Roth GmbH & Co. KG, Karlsruhe, Germany), pure Grindsted^{®} CITREM ester ('BCFS'; here, GRINDSTED^{®} CITREM BC-FS SG by DuPont Nutrition Biosciences ApS, Denmark), or blends of said components in various weight ratios ranging from 30:70 to 95:5 were prepared by mixing the two components and melting them at 90-100 °C to obtain a homogenous coating composition. Using a Ventilus^{®}V-2.5 lab-scale fluidized-bed coater equipped with a Romaco Innojet Hot-Melt-Device IHD 1 (Romaco Innojet, Steinen, Germany), an amount of 225 gram of the ASA-powder were then directly coated with said molten compositions, without any pre-granulation steps and without the use of added solvents.

The following process parameters were used during the hot-melt coating process:

| | |
|---|---|
| - inlet air flow rate: | about 35 m³/h, |
| - inlet air temperature: | about 45 °C for pure PA-coatings, or about 38 °C for BCFS-containing coatings, |
| - atomizing air pressure: | about 1 bar, |
| - atomizing air temperature: | 90 °C, |
| - nozzle temperature: | 90 °C, |
| - spray-rate: | about 5 g/min, and |
| - product temperature: | about 43 °C. |

The coating levels, based on the final weight of the pH-dependent release particle, thus obtained were 25 % or higher; **Fig. 1-4**, for instance, depict release results of the ASA-batches obtained by the above process at coating levels of 32 % or 50 %.

Applying 50 % coating level required a spraying time of about 45 min, and a total processing time of about 55 min, which is significantly shorter (on average, at least 3x shorter) than spraying the same amount of coating material from an aqueous dispersion.

### Example 2 - Coating of acetylsalicylic acid (ASA) or N-acetylcysteine (NAC) drug crystals

Acetylsalicylic acid (ASA) drug crystals with a median particle size D50 of 176.5 µm, as determined by dynamic image analysis using aCamsizer^{®} XT device (Retsch Technology GmbH, Germany, or alternatively N-acetylcysteine (NAC) drug crystals with a median particle size (D50)of 225.6 µm, were used as the core materials. As mentioned above, the ASA-drug crystals exhibit an oblong, needle-like shape, while the NAC-drug crystals exhibit a rounder, more elliptical, 'potato-like' shape.

Similar to Example 1, the two drug materials were used 'as is', with no added steps being taken prior to the hot-melt coating step to formulate them into drug cores. In other words, the cores of the coated particles obtained from the process of this example essentially consist of ASA or NAC, respectively.

Molten coating compositions consisting of palmitic acid (PA) and Grindsted^{®} CITREM ester ('BCFS') in various ratios ranging from 45:55 to 90:10 were prepared by mixing the two components and melting them at 90 °C to obtain a homogenous coating composition.

In a method similar to the one of Example 1 but using an inlet air flow rate of 30 m³/h, the molten compositions were applied to the core materials.

The coating levels, based on the final weight of the pH-dependent release particle, thus obtained were 25 % or higher; **Fig. 5**, for instance, depicts release results of the ASA-batches obtained by the above process at coating levels of 50 %; **Fig. 6** and **Fig. 7** depict release results of the NAC-batches obtained by the above process at coating levels of 50 %, 55 %, 60 % or 70 %.

Applying 50 % coating level required a spraying time of about 45 min, and a total processing time of about 55 min, which is significantly shorter (on average, at least 3x shorter) than spraying the same amount of coating material from an aqueous dispersion.

### Example 3 - Drug release studies

Following method A of the European Pharmacopoeia monography Ph. Eur. 2.9.3. (Ph. Eur., 10th edition, 2020), the in vitro drug release of the hot-melt coated particles of Examples 1 to 2 was determined in 750 mL 0.1 N hydrochloric acid (HCl; pH 1.0) for the first 2 hours using a USP type 2 dissolution apparatus (paddle stirrer) at 100 rpm and 37 ± 0.5 °C (acid stage). This was immediately followed by the buffer stage of the release test, which was achieved by adding to the acidic release medium within 5 minutes 250 mL trisodium phosphate dodecahydrate buffer solution - preheated to 37 ± 0.5 °C and comprising 0.2 vol.-% of polysorbate 80 (based on the 1000 mL volume of the resulting release medium) - so as to shift the pH of the release medium to pH 6.8. The polysorbate addition to the pH 6.8 buffer was chosen as a means to mimic the surface tension of biorelevant media such as fasted-state simulated intestinal fluid (FaSSIF). Since polysorbate 80 degrades in 0.1 N HCl, though, no respective addition was made during the acid stage. The drug release during the buffer stage was then measured for at least another 0.75 h, typically for another 2 h.

The drug release from the hot-melt coated particles (in percent of the total drug content of the particles) was determined using UV-spectroscopy; the accuracy and reliability of the UV-quantification method were reconfirmed using HPLC-quantification. The results of the drug release studies are depicted in **Fig. 1** to **7** and discussed in more detail in the description above.

The following list of numbered items are embodiments comprised by the present invention:
1. A hot-melt coated pH-dependent-release particle comprising a core and a coating,
   wherein the core comprises, or consists of, an active ingredient, and
   wherein the coating comprises, or consists of, a fatty acid which is solid at room temperature, and a CITREM ester; and
   wherein the pH-dependent release refers to a dissolution profile - as determined in an aqueous medium at 37 °C using a USP Dissolution Apparatus type 2 (paddle apparatus) at a stirring speed of 100 rpm - in which:
   - during the initial acid stage in 750 mL 0.1 N hydrochloric acid, no more than 25 % of the active ingredient contained in the particle are released within 2 h, and
   - during the buffer stage in 1000 mL pH 6.8 phosphate buffer, at least 75 % of the active ingredient contained in the particle is released within no more than 2 h in said pH 6.8 phosphate buffer.
2. The particle of item 1, wherein the pH-dependent release refers to a dissolution profile - as determined in an aqueous medium at 37 °C using a USP Dissolution Apparatus type 2 (paddle apparatus) at a stirring speed of 100 rpm - in which:
   - during the initial acid stage in 750 mL 0.1 N hydrochloric acid, no more than 20 % of the active ingredient contained in the particle are released within 2 h, and
   - during the buffer stage in 1000 mL pH 6.8 phosphate buffer, at least 75 % of the active ingredient contained in the particle is released within no more than 1.5 h in said pH 6.8 phosphate buffer.
3. The particle of any one of the preceding items, wherein the pH-dependent release refers to a dissolution profile - as determined in an aqueous medium at 37 °C using a USP Dissolution Apparatus type 2 (paddle apparatus) at a stirring speed of 100 rpm - in which:
   - during the initial acid stage in 0.1 N hydrochloric acid, no more than 15 % of the active ingredient contained in the particle are released within 2 h, and
   - during the buffer stage in 1000 mL pH 6.8 phosphate buffer, at least 75 % of the active ingredient contained in the particle is released within no more than 1 h in said pH 6.8 phosphate buffer.
4. The particle of any one of the preceding items, wherein the pH-dependent release refers to a dissolution profile - as determined in an aqueous medium at 37 °C and using a USP Dissolution Apparatus type 2 (paddle apparatus) at a stirring speed of 100 rpm - in which:
   - during the initial acid stage in 0.1 N hydrochloric acid, less than 10 % of the active ingredient contained in the particle are released within 2 h, and
   - during the buffer stage in 1000 mL pH 6.8 phosphate buffer, at least 75 %, preferably at least 80 %, and further preferably at least 85 %, of the active ingredient contained in the particle is released within no more than 0.75 h in said pH 6.8 phosphate buffer.
5. The particle of any one of the preceding items, wherein the pH-dependent release refers to a dissolution profile - as determined in an aqueous medium at 37 °C and using a USP Dissolution Apparatus type 2 (paddle apparatus) at a stirring speed of 100 rpm - in which:
   - during the initial acid stage in 0.1 N hydrochloric acid, less than 10 % of the active ingredient contained in the particle are released within 2 h, and
   - during the buffer stage in 1000 mL pH 6.8 phosphate buffer, at least 75 % of the active ingredient contained in the particle is released within no more than 1.5 h, preferably within no more than 1 h, in said pH 6.8 phosphate buffer.
6. The particle of any one of the preceding items, wherein, during the buffer stage,
   a surfactant is added to at least the pH 6.8 phosphate buffer, optionally at
   a concentration of 0.01 to 0.5 vol.-% based on the volume of the pH 6.8 phosphate buffer.
7. The particle of any one of item 6, wherein the surfactant is a polysorbate, optionally polysorbate 80.
8. The particle of any one of the preceding items, wherein, during the buffer stage, 0.2 vol.-% of polysorbate 80 is added to the 1000 mL of pH 6.8 phosphate buffer.
9. The particle of any one of the preceding items, wherein the coating comprises at least 2 % of the CITREM ester, preferably at least 3 %, more preferably at least 5 %.
10. The particle of any one of the preceding items, wherein the weight ratio of the fatty acid and the CITREM ester is in the range of 45:55 to 98:2, or 50:50 to 97:3, or 60:40 to 95:5, or 70:30 to 90:10.
11. The particle of any one of the preceding items, wherein the fatty acid is a C₁₃-C₂₂ fatty acid, a saturated fatty acid, or a saturated C₁₃-C₂₂ fatty acid.
12. The particle of any one of the preceding items, wherein the fatty acid has a melting point in the range of 55-75 °C, or from 60-70 °C.
13. The particle of any one of the preceding items, wherein the fatty acid is palmitic acid and/or stearic acid.
14. The particle of any one of the preceding items, wherein the fatty acid is palmitic acid.
15. The particle of any one of the preceding items, wherein the CITREM ester is a partially neutralized citric acid ester of mono- and diglycerides (E472c) combined with mono- and diglycerides (E471).
16. The particle of any one of the preceding items, wherein the coating is substantially free from water-swellable polymers, in particular, polysaccharides, such as cellulosic polymers (e.g., cellulose ethers), alginic acid derivatives (e.g., alginates), or polyacrylate derivatives.
17. The particle of any one of the preceding items, wherein the coating essentially consists of the fatty acid and the CITREM ester.
18. The particle of any one of the preceding items, wherein the coating essentially consists of palmitic acid and, as the CITREM ester, a partially neutralized citric acid ester of mono- and diglycerides (E472c) combined with mono- and diglycerides (E471).
19. The particle of any one of the preceding items, wherein the coating essentially consists of palmitic acid and, as the CITREM ester, a partially neutralized citric acid ester of mono- and diglycerides (E472c) combined with mono- and diglycerides (E471); and wherein the coating comprises at least 2 % of this CITREM ester, preferably at least 3 %, more preferably at least 5 %.
20. The particle of any one of the preceding items, wherein the coating essentially consists of palmitic acid and, as the CITREM ester, a partially neutralized citric acid ester of mono- and diglycerides (E472c) combined with mono- and diglycerides (E471); and wherein the weight ratio of the fatty acid and this CITREM ester is in the range of 45:55 to 98:2, or 50:50 to 97:3, or 60:40 to 95:5, or 70:30 to 90:10.
21. The particle of any one of the preceding items, wherein the coating level, based on the final weight of the pH-dependent release particle, is 25 % or higher, or 30 % or higher, or 32 % or higher, 35 % or higher.
22. The particle of any one of the preceding items, wherein the coating level, based on the final weight of the pH-dependent release particle, is in the range of 25-70 %, or in the range of 30-70 %, or in the range of 30-65 %, or in the range of 35-60 %.
23. The particle of any one of the preceding items, wherein the coating level, based on the final weight of the pH-dependent release particle, is 32 %, 40 %, 50 %, 55 %, or 60 %.
24. The particle of any one of the preceding items, wherein the active ingredient is selected from the group consisting of acid-sensitive drugs, drugs sensitive to enzymatic degradation, gastro-irritant drugs, poorly tasting drugs, and/or drugs with an absorption window in the small intestine.
25. The particle of any one of the preceding items, wherein the active ingredient is selected from the group consisting of proton-pump inhibitors, analgesics such as non-steroidal anti-inflammatory drugs (NSAIDs), bisphosphonates, colitis drugs, fumaric acid compounds, glucocorticoids, antidepressants, expectorants, enzymes, vitamins, mineral salts, plant extracts, omega-3 fatty acids, antioxidants, and probiotics.
26. The particle of any one of the preceding items, wherein the active ingredient is a non-steroidal anti-inflammatory drug (NSAID), e.g., acetylsalicylic acid.
27. The particle of any one of the preceding items, wherein the active ingredient is an expectorant, e.g., N-acetylcysteine.
28. The particle of any one of the preceding items, wherein the active ingredient exhibits a median particle size (D50) in the range of 100 µm to 300 µm, or 125 µm to 275 µm, or 150 µm to 250 µm, as measured by dynamic image analysis.
29. The particle of any one of the preceding items, wherein the core of the coated particle contains more than 50 %, or more than 60 %, or more than 70 %, or more than 80 %, or more than 90 %, or more than 95 % of the active ingredient, based on the weight of the core.
30. The particle of any one of the preceding items, wherein the core of the coated particle consists of the active ingredient.
31. The particle of any one of the preceding items, wherein the particle is storage stable.
32. The particle of any one of the preceding items, wherein the particle exhibits a median particle size (D50) in the range of 200 µm to 400 µm, or 225 µm to 375 µm, or 250 µm to 350 µm, as measured by dynamic image analysis.
33. The particle of any one of the preceding items, wherein the particle is orally ingestible.
34. A pharmaceutical or nutraceutical composition comprising the particle of any of items 1 to 33, or a plurality thereof.
35. The composition of item 34, being orally ingestible; for instance, formulated as (i) granules, such as dispersible granules, effervescent granules, direct-to-mouth granules; or as (ii) tablets, such as dispersible tablets, effervescent tablets, or orally disintegrating tablets; or as (iii) granules filled into capsules.
36. A method for the preparation of the hot-melt coated pH-dependent release particle of any of items 1 to 33, comprising the steps of:
   (a) providing a core particle comprising, or consisting of, an active ingredient,
   (b) providing a coating composition that comprises, or consists of, a fatty acid which is solid at room temperature, and a CITREM ester,
   (c) melting the coating composition,
   (d) coating the core particle with the molten coating composition of step (c),
   (e) cooling the product of step (d) to allow the molten composition to solidify and form the hot-melt coated pH-dependent release particle.
37. The method of item 36, wherein the molten coating composition of step (c) is applied in step (d) by pouring, dripping, and/or spraying it into, or onto, the core particles provided in step (a).
38. The method of items 36 or 37, wherein the core particles provided in step (a) are spray-coated with the molten coating composition of step (c).
39. The method of any one of items 36 to 38, wherein the provided core particles of step (a) are coated in a fluidized-bed coater.
40. The method of any one of items 36 to 39, wherein the temperature of the inlet air during step (d) is in the range of 30 °C to 50 °C; or in the range of 35 °C to 48 °C; or in the range of 38 °C to 45 °C.
41. The method of any one of items 36 to 40, wherein the spray-rate of the molten coating composition during step (d) per kilogram of to-be-coated product - for instance, the spray-rate chosen for spraying the molten coating composition in(to) a fluidized-bed coater - is in the range of about 13 to 31 g/kg/min; or in the range of about 17 to 27 g/kg/min; or in the range of about 20 to 25 g/kg/min, such as about 22 g/kg/min.
42. The method of any one of items 36 to 41, wherein the process is carried out in a lab-scale fluidized-bed coater (e.g., a Ventilus^{®}V-2.5), and the spray-rate of the molten coating composition during step (d) is in the range of about 3.0 to 7.0 g/min; or in the range of about 4.0 to 6.0 g/min; or in the range of about 4.5 to 5.5 g/min, such as about 5.0 g/min.

## Claims

1. A hot-melt coated pH-dependent-release particle comprising a core and a coating,
wherein the core comprises, or consists of, an active ingredient, and
wherein the coating comprises, or consists of, a fatty acid which is solid at room temperature, and a CITREM ester; and
wherein the pH-dependent release refers to a dissolution profile - as determined in an aqueous medium at 37 °C using a USP Dissolution Apparatus type 2 (paddle apparatus) at a stirring speed of 100 rpm - in which:
- during the initial acid stage in 750 mL 0.1 N hydrochloric acid, no more than 25 % of the active ingredient contained in the particle are released within 2 h, and
- during the basic stage in 1000 mL pH 6.8 phosphate buffer, at least 75 % of the active ingredient contained in the particle is released within no more than 2 h in said pH 6.8 phosphate buffer.

2. The particle of claim 1, wherein the coating comprises at least 2 wt.-% of the CITREM ester, preferably at least 3 wt.-%, more preferably at least 5 wt.-%.

3. The particle of claims 1 or 2, wherein the weight ratio of the fatty acid and the CITREM ester is in the range of 45:55 to 98:2, or 50:50 to 97:3, or 60:40 to 95:5, or 70:30 to 90:10.

4. The particle of any one of the preceding claims, wherein the fatty acid is a C₁₃-C₂₂ fatty acid, a saturated fatty acid, or a saturated C₁₃-C₂₂ fatty acid.

5. The particle of any one of the preceding claims, wherein the fatty acid has a melting point in the range of 55-75 °C, or from 60-70 °C.

6. The particle of any one of the preceding claims, wherein the fatty acid is palmitic acid and/or stearic acid.

7. The particle of any one of the preceding claims, wherein the CITREM ester is a partially neutralized citric acid ester of mono- and diglycerides (E472c) combined with mono- and diglycerides (E471).

8. The particle of any one of the preceding items, wherein the coating is substantially free from water-swellable polymers, in particular, polysaccharides, such as cellulosic polymers, alginic acid derivatives, or polyacrylate derivatives.

9. The particle of any one of the preceding claims, wherein the coating essentially consists of the fatty acid and the CITREM ester.

10. The particle of any one of the preceding claims, wherein the coating level, based on the final weight of the pH-dependent release particle, is 25 wt.-% or higher, or 30 wt.-% or higher, 35 wt.-% or higher.

11. The particle of any one of the preceding items, wherein the coating level, based on the final weight of the pH-dependent release particle, is in the range of 25-70 %, or in the range of 30-70 %, or in the range of 30-65 %, or in the range of 35-60 %.

12. The particle of any one of the preceding claims, wherein the active ingredient is selected from the group of acid-labile drugs, gastro-irritant drugs, and/or drugs with an absorption window in the small intestine.

13. The particle of any one of the preceding claims, wherein the core consists of the active ingredient.

14. A pharmaceutical or nutraceutical composition comprising the particle of any of claims 1 to 13, or a plurality thereof.

15. A method for the preparation of the hot-melt coated pH-dependent release particle of any of claims 1 to 13, comprising the steps of:
(f) providing a core particle comprising, or consisting of, an active ingredient,
(g) providing a coating composition that comprises, or consists of, a fatty acid which is solid at room temperature, and a CITREM ester,
(h) melting the coating composition,
(i) coating the core particle with the molten coating composition of step (c),
(j) cooling the product of step (d) to allow the molten composition to solidify and form the hot-melt coated pH-dependent release particle.
